# EUROPÄISCHE PATENTSCHRIFT

(11) **EP 4 491 208 B1**
(45) Veröffentlichungstag und Bekanntmachung des Hinweises auf die Patenterteilung: **18.11.2026**
(21) Anmeldenummer: 23184684.1
(22) Anmeldetag: 11.07.2023
(51) Int. Cl.: A61M 5/32, A61M 5/34, A61M 5/50, A61M 5/315, A61M 5/31, A61M 5/24

(54) **MEDIZINISCHE SPRITZE UND NADELAUFSATZ FÜR EINE SOLCHE SPRITZE**
MEDICAL SYRINGE AND NEEDLE CAP FOR SUCH A SYRINGE
SERINGUE MÉDICALE ET EMBOUT D'AIGUILLE POUR UNE TELLE SERINGUE

(43) Veröffentlichungstag der Anmeldung: 15.01.2025
(73) Patentinhaber: Inductio AG, 6052 Hergiswil (CH)
(72) Erfinder: KOLLER, Horst, 8730 Uznach (CH)
(74) Vertreter: Tergau & Walkenhorst Intellectual Property GmbH

(56) Entgegenhaltungen:
- EP-A1- 3 738 629
- WO-A1-2013/146099
- US-A- 3 500 830
- US-A1- 2012 083 744

## Beschreibung

Die Erfindung bezieht sich auf eine medizinische Spritze mit einem einen Wirkstoffcontainer aufweisenden Spritzenkörper, wobei der Wirkstoffcontainer mittels einer im Bereich des distalen Endes des Spritzenkörpers in diesem angeordneten, durchstechbaren Membran dichtend verschlossen ist, und mit einem am Spritzenkörper anbringbaren Nadelaufsatz, der ein von einer Injektionsnadel und einer mit dieser fluidseitig verbundenen Septumnadel gebildetes Nadelsystem trägt. Sie betrifft weiter einen Nadelaufsatz für eine solche medizinische Spritze.

Solche Spritzen werden üblicherweise auch als "Trockennadel-System" bezeichnet. Sie dienen dazu, mit einem Wirkstoff vorbefüllte Spritzen in einem weitgehend vormontierten System bereitzustellen, wobei aus Gründen der Lagerfähigkeit und auch des Schutzes des Wirkstoffs vor eingetragenen Kontaminationen während der Lagerzeit und auch während der Handhabung die Spritzennadel separat vom Wirkstoff gehalten werden und insbesondere nicht mit diesem in Kontakt kommen soll. Eine so genannte "trockene" Spritzennadel liegt dann vor, wenn die Spritzennadel während der Lagerung oder sonstiger Handhabung nicht vom Wirkstoff benetzt wird oder auf sonstige Weise mit diesem in Kontakt kommt. Um dies zu ermöglichen, wird das Wirkstoffreservoir in einer solchen Spritze üblicherweise mit einer durchstechbaren Membran abgedichtet und versiegelt. Diese auch als "Septum" bezeichnete Membran wird zur Verabreichung des Wirkstoffs von einer zugeordneten Septumnadel durchstochen, die ihrerseits fluidseitig mit der eigentlichen Injektionsnadel in Verbindung steht und somit die fluidseitige Verbindung der Injektionsnadel mit dem Wirkstoffreservoir herstellen kann.

Im allgemeinen werden vorgefüllte Spritzen aus Glas oder Kunststoff zur Verwendung und Verabreichung der bereits in der Spritze befindlichen und gebrauchsfertigen Arzneimittelformulierung geliefert. Sie sind im Allgemeinen mit einem Luftraum oder einer Gasblase gefüllt, da sie vom offenen Ende gegenüber dem Nadelende aus befüllt und dann verschlossen werden. Gasblasen können dazu führen, dass sich der Stopfen bewegt und die Sterilität während des Transports aufgrund von Druckschwankungen verloren geht. Außerdem kann sich das Medikament durch den Kopfraum bewegen und das Silikonöl von der Oberfläche ablösen. Silikonöltröpfchen können außerdem mit Arzneimittelmolekülen und insbesondere mit biologischen Wirkstoffen in Wechselwirkung treten. Vorgefüllte Spritzen können mit Hilfe von Vakuumverschlüssen blasenfrei befüllt werden. Dies ist jedoch im Allgemeinen auf niedrigviskose Formulierungen beschränkt und verlangsamt die Abfüllanlage erheblich, was zusätzliche Kosten verursacht.

Wenn möglich, sollten die gefüllten Spritzen mit dem eingefüllten Arzneimittel gassterilisiert werden. Vorgefüllte Spritzen mit gestochener Nadel, ob aus Glas oder Kunststoff, können in der Regel nicht endsterilisiert werden, da der Nadelschutz, der die Nadelspitze verschließt, aus durchlässigen Gummimischungen besteht, damit die Nadel vor der Abfüllung in der Produktion gassterilisiert werden kann. Eine endgültige Sterilisation durch Gas würde das Medikament zersetzen. Vorgefüllte Luer-Lock-Spritzen können zwar endsterilisiert werden, doch muss der Benutzer die Spritze vor dem Aufsetzen der Nadel aus der Sterilverpackung nehmen, was zu einem Verlust der Sterilität führen kann.

Die endgültige Sterilisation einer vorgefüllten Spritze ist in den meisten Fällen ein großer Vorteil, da sie das Risiko einer Kreuzkontamination und eines Verlusts der Sterilität erheblich verringert. So werden beispielsweise Luer-Lock-Spritzen in der Augenheilkunde verwendet, wo die Spritze vorzugsweise endsterilisiert wird. Allerdings muss der Anwender die Nadel nach der Entnahme der Spritze aus der sterilen Schutzblase noch anbringen, was zu möglichen Anwenderfehlern und dem Risiko eines Sterilitätsverlustes führt.

Eine Alternative zu vorgefüllten Spritzen sind Spritzen oder Pens auf Kartuschenbasis. Die Kartusche besteht aus einem Glas- oder Kunststoffzylinder, einem durchstechbaren Septum, das an einem Ende von einer gebördelten Metallhülse gehalten wird, und einem Stopfen/Kolben am anderen Ende. In der Regel wird eine Injektionsnadel vom Benutzer vor der Verwendung manuell angebracht, indem eine doppelendige Nadel auf einen Patronenhalter oder eine andere Vorrichtung wie einen Stift, der die Patrone hält, geschraubt wird. Dadurch wird die Membran perforiert oder durchstochen und ein Flüssigkeitsweg zwischen dem Arzneimittelinhalt der Patrone und der Injektionsnadel geschaffen. Solche Anordnungen werden typischerweise in Pen-Injektoren wie Insulin-Pens und Zahnarztsystemen mit Patronen verwendet. Da der Benutzer die Nadel anbringen muss, besteht die Gefahr von Bedienungsfehlern und des Verlusts der Sterilität.

Bei den genannten Trocken- oder Doppelnadelsystemen für vorbefüllte Spritzen ist von erheblicher Bedeutung, dass die Septumnadel zwar bei der Bereitstellung der Spritze für die Applikation des Wirkstoffs das die Membran zuverlässig durchstößt, so dass die für die Verabreichung des Wirkstoffs erforderliche fluidseitige Verbindung des Wirkstoffreservoirs mit der Injektionsnadel hergestellt werden kann. Andererseits ist aber sicherzustellen, dass die Septumnadel während Transport, Lagerung oder sonstiger Handhabung zuverlässig vom Septum beabstandet gehalten wird, so dass dieses durch die Septumnadel nicht unbeabsichtigt beschädigt werden kann und damit der Wirkstoff kontaminiert werden könnte oder Wirkstoffverluste auftreten könnten.

Medizinische Spritzen mit aufschraubbarem Nadelaufsatz mit einem solchen Doppelnadelsystem sind beispielsweise bekannt aus der US 3,500,830 oder aus der WO 2013146099 A1.

Der Erfindung liegt daher die Aufgabe zugrunde, eine medizinische Spritze der oben genannten Art anzugeben, mit der die genannten Erfordernisse zuverlässig, aber auch auf besonders einfache Weise und somit auch für die Nutzung in großen Stückzahlen geeignet erfüllt werden können. Des Weiteren soll ein hierfür besonders geeigneter Nadelaufsatz für eine solche medizinische Spritze angegeben werden.

Bezüglich des Nadelaufsatzes für eine medizinische Spritze wird diese Aufgabe erfindungsgemäß gelöst mit den Merkmalen des Anspruchs 1. Bezüglich der medizinischen Spritze wird diese Aufgabe gelöst mit den Merkmalen des Anspruchs 2.

Die Erfindung geht dabei von der Überlegung aus, dass zur besonders zuverlässigen Einhaltung der genannten Erfordernisse eine zuverlässige und sehr präzise einstellbare Führung der Septumnadel in Relation zur durchstechbaren Membran bereitgestellt werden sollte. Gemäß einem Aspekt der Erfindung wird hierzu die Übersetzung einer Drehbewegung in eine Vorschubbewegung mittels einer Gewindeverbindung angesehen, mit der, je nach Wahl und Auslegung der Gewindeparameter, ein sehr stabiler, mechanisch gut geführter und sehr fein dosierbarer Vortrieb der Septumnadel auf die Membran zu erzeugt werden kann. Ein solche Gewindeantrieb kann zudem auch selbsthemmend ausgeführt sein, so dass im noch nicht aktiven Zustand, also während der Lagerung oder sonstiger Handhabung, die Septumnadel zuverlässig und beabstandet von der Membran positioniert werden kann. Auf dieser Basis ist für die Spritze für die Anbringung des Nadelaufsatzes am Spritzenkörper eine Schraubverbindung vorgesehen, mittels derer die Septumnadel besonders präzise positioniert und bewegt werden kann.

Vorteilhafte Ausgestaltungen der Erfindung sind Gegenstand der Unteransprüche.

Vorteilhafterweise und gemäß einem Aspekt der Erfindung bewirkt das Einschrauben des Nadelaufsatzes in den Spritzenkörper einen Vortrieb der Septumnadel in Richtung hin zur durchstechbaren Membran, wobei die Eigenschaften dieses Vortriebs mittels der Gewindeparameter der Schraubverbindung, insbesondere der Ganghöhe des Gewindes, besonders präzise eingestellt werden können.

In weiterer vorteilhafter Ausgestaltung ist die Schraubverbindung dabei als Luer-Verbindung ausgestaltet. Dabei wird insbesondere der Erkenntnis Rechnung getragen, dass Luer-Verbindung im Bereich derartiger Applikationen grundsätzlich weit verbreitet sind, so dass im Sinne eines Plattformgedankens und der Austauschbarkeit und Kompatibilität verschiedenartiger Komponenten miteinander hier an vorhandene Bauteile angeknüpft werden könnte.

Hinsichtlich der Parameterwahl sind gemäß einem bevorzugten Aspekt der Erfindung die Komponenten derart dimensioniert, dass die Septumnadel die im Spritzenkörper angeordnete Membran durchsticht, wenn die Schraubverbindung vollständig hergestellt ist. Dies bedeutet, dass die Komponenten der Schraubverbindung, wenn diese vollständig ineinander eingeschraubt sind und dementsprechend auf Endanschlag gehen, die Septumnadel zuverlässig die fluidseitige Verbindung zum Inneren des Wirkstoffreservoirs herstellt.

In vorteilhafter Ausgestaltung der Erfindung wird die vorgesehene Schraubverbindung durch ein an einem Grundkörper des Nadelaufsatzes angeordnetes Außengewinde in Kombination mit einem an dieses angepassten Innengewinde eines Anschlussstutzens des Spritzenkörpers gebildet.

In einem besonders vorteilhaften und eigenständig erfinderischen Aspekt der Erfindung ist die Schraubverbindung zudem dafür ausgestaltet, während der Lager- oder Transportphase die Septumnadel sicher in einer ausreichenden Entfernung von der Membran zu positionieren und dort zu fixieren, so dass ein unerwünschtes Durchstechen der Membran oder auch nur deren Beschädigung durch die Septumnadel sicher ausgeschlossen ist. Dazu kann gemäß einem Aspekt der Erfindung die Schraubverbindung mit einem entfernbaren Zwischenanschlag ausgestattet sein, der während der Lager- oder Transportphase nur ein teilweises Einschrauben der Komponenten und somit nur einen teilweisen Vorschub der Septumnadel auf die Membran zu erlaubt, wobei diese Bewegung durch den Zwischenanschlag gemäß einem Aspekt der Erfindung derart frühzeitig begrenzt wird, dass die Septumnadel die Membran sicher nicht erreicht und ausreichend beabstandet von dieser in ihrer Position fixiert wird. Erst nach Entfernen dieses Zwischenanschlags soll ein vollständiger Vorschub der Septumnadel ermöglicht werden, bei dem diese die Membran erreichen kann und letztlich durchsticht.

Um dies zu ermöglichen und auf besonders einfache Weise und für eine Massenproduktion geeignet bereitzustellen, umfasst gemäß einem Aspekt der Erfindung der Nadelaufsatz eine Nadelschutzkappe, die mittels einer endseitig angeformten Abdeckschürze das Außengewinde teilweise umgibt. Die Abdeckschürze ist dabei vorzugsweise derart angeordnet und dimensioniert, dass, wenn ihr endseitiger Rand an der Stirnfläche des Anschlussstutzens anliegt, die Septumnadel noch beabstandet von der Membran im Spritzenkörper ist. Solange sich die Nadelschutzkappe dann auf dem Nadelaufsatz befindet, ist ein weiteres Einschrauben der Komponenten durch den so gebildeten Anschlag nicht möglich und damit ausgeschlossen; ein versehentliches oder unbeabsichtigtes Beschädigen oder Durchstechen der Membran durch die Septumnadel ist damit ausgeschlossen.

Ein Ausführungsbeispiel der Erfindung wird anhand einer Zeichnung näher erläutert. Darin zeigen:
- FIG. 1: Die Komponenten einer mit einem Wirkstoff vorbefüllten medizinischen Spritze im Längsschnitt in Explosionsdarstellung,
- FIG. 2: den Spitzenkörper der Spritze nach FIG. 1 vergrößert im Längsschnitt,
- FIG. 3: den Nadelaufsatz der Spritze nach FIG. 1 in perspektivischer Ansicht,
- FIG. 4: den Nadelaufsatz nach FIG. 3 vergrößert im Längsschnitt,
- FIG. 5: die aus den Komponenten zusammengesetzte Spritze nach FIG. 1 im Längsschnitt im lagerfähigen Zustand,
- FIG. 6: eine ausschnittsweise Vergrößerung von FIG. 5,
- FIG. 7: die Spritze nach FIG. 5 nach Entfernen der Nadelschutzkappe und vor der Bereitstellung zur Verwendung im Längsschnitt, und
- FIG. 8: die Spritze nach FIG. 5 mit vollständig aufgeschraubtem Nadelaufsatz im Längsschnitt.

Gleiche Teile sind in allen Figuren mit denselben Bezugszeichen versehen.

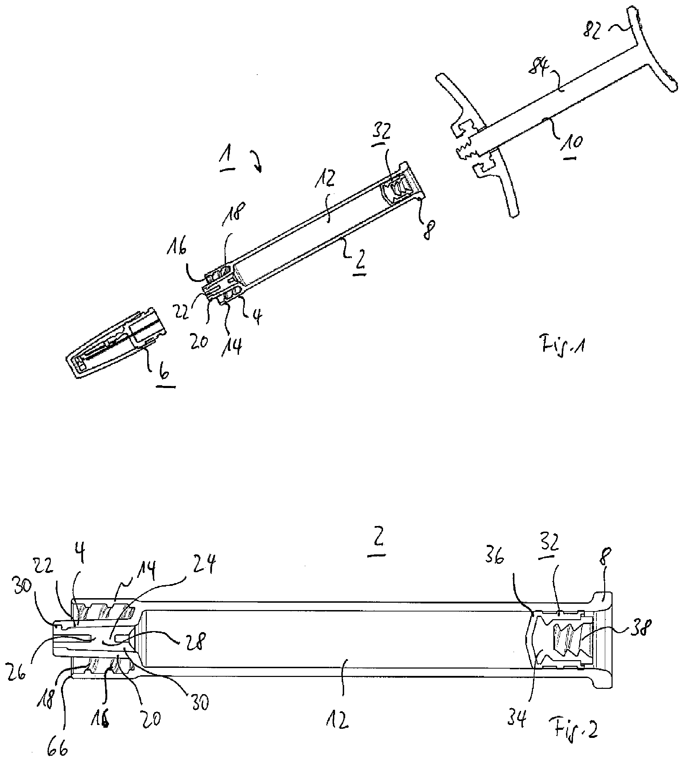

Die medizinische Spritze 1 gemäß FIG. 1 umfasst im Wesentlichen drei Baugruppen oder Komponenten, nämlich einen den Spritzenkörper 2 bildenden Wirkstoffbehälter (auch als Kartuschen- oder Patroneneinheit bezeichnet), der einerseits an seinem distalen Ende 4 zur Verbindung mit einem Nadelaufsatz 6 oder Nadelhalter und andererseits an seinem proximalen Ende 8 zur Verbindung mit einem Betätigungselement 10 vorgesehen ist. Der Spritzenkörper 2 ist entsprechend einer herkömmlichen Bauweise zylindrisch oder rohrförmig als Hohlkörper 12 ausgeführt, der im Ausführungsbeispiel direkt zur Aufnahme des medizinischen Wirkstoffs und somit als Wirkstoffcontainer vorgesehen ist. Alternativ könnte der Wirkstoffcontainer aber auch in der Art einer so genannten Kartusche oder Ampulle als separates Bauteil ausgeführt sein, das in das eigentliche Spritzengehäuse einschiebbar oder einlegbar sein kann.

Die Spritze 1 ist als vorbefüllte medizinische Spritze ausgeführt, die in einem zentralen Abfüllprozess herstellerseitig mit dem Wirkstoff befüllt wird und anschließend mit dem enthaltenen Wirkstoff transportiert und/oder gelagert werden kann, bevor sie für die eigentliche Anwendung, also die Injektion des enthaltenen Wirkstoffs, bereitgestellt wird. Um dabei die entsprechenden Erfordernisse für eine sichere Lagerung und Handhabung, ggf. auch über einen längeren Zeitraum hinweg, erfüllen zu können, ist der den Wirkstoffcontainer bildende Hohlkörper 12 geeignet versiegelt und abgedichtet ausgeführt. Damit soll insbesondere während Transport oder Lagerung ein unerwünschter Wirkstoffverlust ebenso vermieden werden wie ein möglicher Eintrag von Verunreinigungen oder Kontaminationen in den vorgehaltenen Wirkstoff hinein vermieden und möglichst ganz ausgeschlossen werden.

Gemäß einem Aspekt der Erfindung ist der Spritzenkörper 12 für einen Anschluss des Nadelaufsatzes 6 mittels einer Schraubverbindung, in als eigenständig erfinderische Ausgestaltung insbesondere mittels einer Luer-Verbindung, ausgestaltet. Dazu ist der Hohlkörper 12, wie insbesondere der vergrößerten Darstellung im Längsschnitt in FIG. 2 deutlich entnehmbar ist, an seinem distalen Ende 4 mit einem Anschlussstutzen 14 für den Nadelhalter 6 versehen, der innenseitig mit einem Luer-Innengewinde 16 versehen ist. Der Anschlussstutzen 14 umgibt dabei in seinem Innenbereich einen unter Freilassung eines umlaufenden Gewinderaums 18 zentral angeordneten Verbindungszapfen 20, der seinerseits mittig einen sich zum distalen Ende 4 hin kontinuierlich, insbesondere konisch, verjüngenden Verbindungskanal 22 aufweist.

Um die genannte zuverlässig abgedichtete Ausführung des Wirkstoffcontainers zu ermöglichen, ist der Hohlkörper 12 an seinem distalen Ende 4 im Bereich des Anschlussstutzens 14 mit einem integrierten, vom der im Nadelhalter 6 angeordneten Spritzennadel durchstechbaren Verschlusskörper 24 versehen. Der Verschlusskörper 24 ist dabei als dreidimensionaler Formkörper ausgeführt. Dieser bildet gemäß einem als eigenständig erfinderisch angesehenen Aspekt in einem proximalen, dem Innenraum des Hohlkörpers 12 zugewandten Zentralbereich 26 eine vergleichsweise dünn ausgeführte, durchstechbare Membran 28, auch als "Septum" bezeichnet, aus. An die Membran 28 ist umlaufend eine trichterartig geformte Fixierschürze 30 angeformt, die in ihrer Außenkontur an die Innenkontur des Verbindungskanals 22 angepasst ist. Der Verschlusskörper 24 ist somit vom proximalen Ende 8 des Hohlkörpers 12 aus in den Verbindungskanal 22 einsteckbar und dort, aufgrund der sich zum distalen Ende 4 hin verjüngenden Formgebung, in Längsrichtung zum distalen Ende 4 hin fixiert. Nach der Befüllung des Hohlkörpers 12 mit Wirkstoff ist dieser von der proximalen Seite des Verschlusskörpers 24 her mit dem Septum 28 in Kontakt, so dass in diesem Zustand auch eine Verschiebung des Verschlusskörpers 24 in proximaler Richtung nicht mehr möglich ist; der Verschlusskörper 24 ist somit in Längsrichtung im Verbindungskanal 22 fixiert. Selbstverständlich können formbedingt auch wandseitige Haftkräfte oder durch Verformungen bedingte Haftkräfte diese Fixierung bewirken oder verstärken, so dass der Verschlusskörper 24 sicher im Verbindungskanal 22 festgelegt ist.

Hinsichtlich der als eigenständig erfinderisch angesehenen Materialwahl für das Spritzengehäuse bzw. den dieses bildenden Hohlkörper 12 ist insbesondere hohen Ansprüchen an die zuverlässige vorübergehende Lagerung des medizinischen Wirkstoffs einhergehend mit einer besonders hohen Sicherheit im Umgang mit den Komponenten Rechnung getragen. Der als zylindrischer Hohlkörper ausgeführte Spritzenkörper 12 kann dabei aus COC, PC oder PP gefertigt sein, ist im gezeigten Ausführungsbeispiel aber in als erfinderisch angesehener Ausgestaltung aus dem Hochleistungskunststoff Cyclo-Olefin-Polymer (COP) gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist. Der Verschlusskörper 24 ist demgegenüber aus dem vergleichsweise weichen TPE gefertigt, so dass bei hoher Dichtigkeit auch das vorgesehene Durchstechen der Spritzennadel problemlos möglich ist.

In als ebenfalls eigenständig erfinderisch angesehener Ausgestaltung ist für die Herstellung des mit dem Verschlusskörper 24 versehenen Hohlkörpers 12 ein 2K-Spritzgussverfahren vorgesehen. Dabei werden die Komponenten in einem einzigen Prozess gemeinsam hergestellt, so dass die vorgesehene verschiebungssichere Positionierung des Verschlusskörpers 24 im Hohlkörper 12 problemlos und auf einfache Weise erreicht werden kann. Der Spritzenkörper 12 ist vorzugsweise im Spritzgießverfahren gefertigt, wobei unter anderem die Formgebung derart erfolgt, dass mögliche Totvolumina im Inneren besonders geringgehalten sind.

Korrespondierend zum Verschlusskörper 24 ist zum sicheren und dichtenden Verschließen des proximalen Endes 8 innerhalb des Hohlkörpers 12 ein verschiebbarer Stopfen 32 vorgesehen. Dieser ist derart dimensioniert, dass er dichtend an der Innenwand des Hohlkörpers 12 anliegt; im Längsbereich des Innenraums des Hohlkörpers 12 zwischen dem Septum 28 und dem Stopfen 32 wird somit das Reservoir für den Wirkstoff ausgebildet, in dem dieser vorgehalten werden kann. Der Stopfen 32 ist dabei in an sich durchaus üblicher Ausgestaltung über das jeweils am proximalen Ende 8 des Spritzenkörpers 12 angeordnete Betätigungselement 10, beispielsweise einen Betätigungsstößel oder auch ein Element mit automatisiertem Antrieb, innerhalb des Hohlkörpers 12 verschiebbar.

Um auch höchsten Ansprüchen hinsichtlich Dichtheit einerseits und Gleiteigenschaften innerhalb des Hohlkörpers 12, beispielsweise hinsichtlich der Losbrechkraft bei Betätigung des Stopfens 32, andererseits Genüge zu tun, ist der Stopfen 32 in als eigenständig erfinderisch angesehener Ausgestaltung mehrkomponentig, insbesondere zweikomponentig, ausgeführt. Im Detail ist der Stopfen 32 gemäß einem Aspekt der Erfindung in der in der nicht vorveröffentlichten Europäischen Patentanmeldung Nr 22206993.2 ausgeführt, deren Offenbarung bezüglich der Ausgestaltung des Stopfens 32 vollumfänglich mit einbezogen wird ("incorporation by reference").

Der gemäß einem Aspekt der Erfindung zweikomponentig oder als "2K-Stopfen" ausgeführte Stopfen 32 umfasst einen zentralen Stopfenkörper 34 aus einem vergleichsweise harten Kunststoff, im Ausführungsbeispiel und gemäß einem Aspekt der Erfindung aus Polypropylen (PP), der von einem aus im Vergleich dazu weicheren Kunststoffmaterial, im Ausführungsbeispiel und gemäß einem Aspekt der Erfindung TPE, gebildeten Dichtmantel 36 umgeben ist. Der zentrale Stopfenkörper 34 dient dabei im Wesentlichen zur Form- und Strukturstabilität und zur Weiterleitung der eingeleiteten Kräfte, insbesondere in Längsrichtung bei der Verschiebung des Stopfens 32 innerhalb des Spritzenkörpers 12. Dazu kann der zentrale Stopfenkörper 34 innenseitig mit einem Aufnahmekanal 38, beispielsweise mit einem Gewinde, versehen sein, über den eine Verbindung mit einem externen Betätigungsmittel, beispielsweise dem Betätigungselement 10 oder dem Betätigungsstößel der Spritze 1, ermöglicht ist.

Gemäß einem weiteren, als unabhängig und eigenständig erfinderisch angesehenen Aspekt der Erfindung ist der Spritzenkörper 12 durch die beschriebene Bauweise gezielt für einen hoch effizienten, für große Stückzahlen geeigneten Herstell- und Abfüllprozess geeignet ausgeführt. Dabei wird zunächst in einem ersten Schritt, wie vorstehend beschrieben, der mit dem Verschlusskörper 24 endseitig verschlossene Hohlkörper 12 als 2K- oder Hypridkomponente hergestellt. Weiterhin wird der Stopfen 32 bereitgestellt. Der Hybridkörper aus Hohlkörper 12 und integriertem Verschlusskörper 24 wird dabei gemäß einem Aspekt der Erfindung unter Einhaltung der Vorgaben und Vorschriften für Primärpackmittel hergestellt, so dass er direkt, ohne Umwege und ohne weitere Zwischenschritte von der Spritzgussanlage zur Abfüll-Anlage überführt werden kann. Die sonst in diesem Zusammenhang notwendigen Zwischenschritte Waschen, Silikonisieren oder Sterilisieren sind damit nicht mehr notwendig, was eine erhebliche Prozessvereinfachung bedeutet.

Bei der Abfüllung, also in der Abfüllanlage, wird der mit dem Verschlusskörper 24 versehene Hohlkörper 12 gemäß einem weiteren Aspekt der Erfindung sodann vertikal, also mit seiner Längsachse parallel zur Erdanziehungskraft, ausgerichtet, und zwar derart, dass das in diesem Zustand noch offene proximale Ende 8 nach "oben" und das mit dem Verschlusskörper 24 verschlossene distale Ende 4 nach "unten", also zur Erde hin, weist. Damit kann die Befüllung des Spritzenkörpers 12 mit dem Wirkstoff beginnen. Dies erfolgt gemäß einem Aspekt der Erfindung in einer Groß-Abfüllanlage, in der eine Vielzahl von Spritzenkörpern parallel und gleichzeitig befüllt werden können. Nach Einbringung der vorgesehenen Wirkstoffmenge in den Hohlkörper 12 wird schließlich der Stopfen 32 in das proximale Ende 8 des Hohlkörpers 12 eingeschoben und verschließt somit den mit dem Wirkstoff befüllten Innenraum. Anschließend ist der befüllte Spitzenkörper 12 für die nachfolgenden Verarbeitungsschritte bereit.

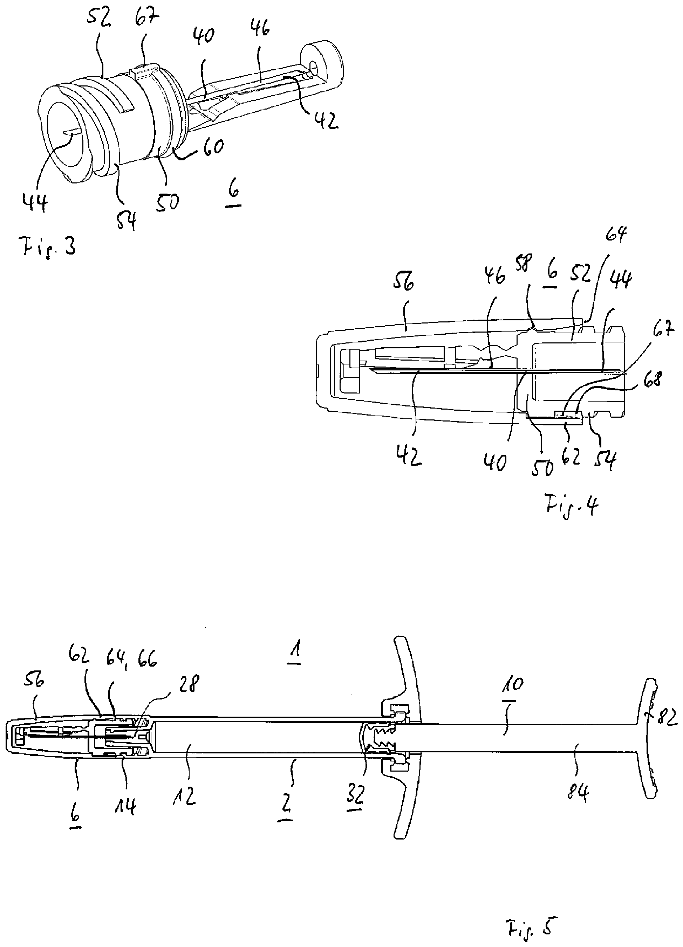

In einem weiteren Verarbeitungsschritt, der vor oder nach der Befüllung des Spritzenkörpers 12 erfolgen kann, wird dieser mit dem Nadelhalter 6 verbunden. Die Spritze 1 ist nämlich für eine Bereitstellung des bereits mit dem Nadelhalter 6 verbundenen Spritzenkörpers 12 für den Verwender vorgesehen; ein Transport und auch die Lagerung der vorbefüllten Spritze 1 ist somit mit bereits angebrachtem Nadelhalter 6 vorgesehen. Um auch dabei auch den höchsten Anforderungen an die Lagerbeständigkeit des Wirkstoffs zu genügen, ist die Spritze 1 als so genanntes Trockennadelsystem ausgeführt, bei dem während der Lagerung oder des Transports ein Kontakt der Spritzennadel mit dem Wirkstoff konsequent vermieden wird. Um dies zu ermöglichen, ist der in FIG. 3 in perspektivischer Ansicht und in FIG. 4 vergrößert im Längsschnitt gezeigte Nadelaufsatz 6 mit einem die Spritzennadel bildenden Nadelsystem 40 versehen, das einerseits eine Injektionsnadel 42 und andererseits eine mit dieser fluidseitig verbundene Septumnadel 44 umfasst.

In für solche Trockennadelsysteme durchaus üblicher Ausgestaltung ist die Injektionsnadel 42 dabei in der Art einer "herkömmlichen" Spritzennadel zum Durchstechen der Haut des Patienten während der Behandlung vorgesehen, wobei die Septumnadel 44 nach "innen hin", also zum Wirkstoffcontainer hin, ausgerichtet ist und zum Durchstoßen des Septums 28 vorgesehen ist. Durch die fluidseitige Verbindung von Injektions- und Septumnadel 42, 44 miteinander wird somit nach dem Durchstechen des Septums 28 eine fluidseitige Verbindung des Innenraums des Wirkstoffcontainers mit der Injektionsnadel 42 hergestellt, so dass der Wirkstoff aus dem Spritzenkörper 2 über die Injektionsnadel 42 ausgebracht werden kann. Im Ausführungsbeispiel, und in durchaus üblicher Ausgestaltung, werden die Injektionsnadel 42 und die Septumnadel 44 von den beiden gegenseitigen Enden einer gemeinsamen Hohlnadel 46 gebildet. Diese Hohlnadel 46 ist in einem den eigentlichen Nadelhalter des Nadelaufsatzes 6 bildenden Grundkörper 50, vorzugsweise gefertigt aus einem harten Kunststoff wie beispielsweise PP, POM oder PC, gelagert und in diesem fixiert.

Bei derartigen Trocken- oder Doppelnadelsystemen ist von erheblicher Bedeutung, dass die Septumnadel 44 zwar bei der Bereitstellung der Spritze 1 für die Applikation des Wirkstoffs das Septum 28 zuverlässig durchstößt, so dass die für die Verabreichung des Wirkstoffs erforderliche fluidseitige Verbindung des Wirkstoffreservoirs mit der Injektionsnadel 42 zuverlässig hergestellt werden kann. Andererseits ist aber sicherzustellen, dass die Septumnadel 44 während Transport, Lagerung oder sonstiger Handhabung zuverlässig vom Septum 28 beabstandet gehalten werden kann, so dass dieses durch die Septumnadel 44 nicht unbeabsichtigt beschädigt werden kann und damit der Wirkstoff kontaminiert werden könnte oder Wirkstoffverluste auftreten könnten.

Gemäß einem als eigenständig erfinderisch angesehenen Aspekt werden diese Erfordernisse mit der Spritze 1 durch eine gezielte Nutzung und Auslegung der für die Anbringung des Nadelaufsatzes 6 am Spritzenkörper 2 vorgesehenen Schraubverbindung erreicht.

Der Nadelaufsatz 6 umfasst zur Herstellung des Schraubanschlusses an seinem Grundkörper 50 gemäß einem Aspekt der Erfindung in einem Anschlussbereich 52 außenseitig ein an das Innengewinde 16 des Spritzenkörpers 2 angepasstes Außengewinde 54, so dass dieses in das Innengewinde 16 einschraubbar ist. Im Ausführungsbeispiel ist gemäß einem Aspekt der Erfindung, korrespondierend zur Ausgestaltung des Innengewindes 16, das Außengewinde 54 als Luer-Außengewinde 54 ausgeführt. Als weitere Komponente ist der Nadelaufsatz 6 zudem gemäß einem Aspekt der Erfindung mit einer auf den Grundkörper 50 des Nadelhalters 6 aufgesteckten Nadelschutzkappe 56 versehen. Die Nadelschutzkappe 56 ist dazu mit einer umlaufenden Innennut 58 versehen, die beim Aufschieben der Nadelschutzkappe 56 auf den Grundkörper 50 mit einer korrespondieren Außenwulst 60 am Grundköper 50 verrastet. Damit wird die Nadelschutzkappe 56 beim Anbringen am Grundkörper 50 in genau definierter Position relativ zum Grundkörper 50 positioniert und dort fixiert. Endseitig umfasst die Nadelschutzkappe 56 zudem eine umlaufende Abdeckschürze 62, die bei ordnungsgemäß positionierter und verrasteter Nadelschutzkappe 56 einen Teil des Luer-Außengewindes 54 außenseitig umschließt und abdeckt.

Bei der Montage des Nadelaufsatzes 6 am Spritzenkörper 12, durch Einschrauben des Außengewindes 54 in das korrespondierende Innengewinde 16 des Spritzenkörpers 2, wird die zentral im Grundkörper 50 gelagerte Hohlnadel 46 mit ihrem die Septumnadel 44 bildenden Ende in den freien Innenkanal innerhalb der Fixierschürze 30 eingebracht, wobei der Vorschub der Septumnadel 44 innerhalb des freien Innenkanals der Fixierschürze 30 durch den Vorschub beim Einschrauben des Außengewindes 54 in das Innengewinde 16 erzeugt wird. Die gewünschte vorläufige Positionierung und Fixierung der Septumnadel 44 in einer Position innerhalb der Fixierschürze 30, bei der sie das Septum 28 noch nicht erreicht oder zumindest noch nicht vollständig durchstößt und dieses somit weder beschädigt noch zerstört, wird dabei gemäß einem als eigenständig erfinderisch angesehenen Aspekt der Erfindung erreicht durch die Auslegung und Dimensionierung der Abdeckschürze 62 der Nadelschutzkappe 56 in Kombination und in Wechselwirkung mit den Komponenten des Spritzenkörpers 2. Beim zunehmenden Einschrauben des Außengewindes 54 in das Innengewinde 16 schlägt nämlich der endseitige Rand 64 der Abdeckschürze 62 an die Stirnfläche 66 des Anschlussstutzens 14 an. Diese bildet somit einen Endanschlag für die Abdeckschürze 62 und somit für das Einschrauben des Nadelaufsatzes 6 insgesamt; ein weiteres Eindrehen ist dann zunächst nicht mehr möglich. Die geometrischen Parameter der genannten Komponenten, also insbesondere der Abdeckbereich der Abdeckschürze 62 im Hinblick auf die Position der Stirnfläche 66 und der Position des Septums 28 innerhalb des Hohlkörpers 12, sind dabei gemäß einem eigenständig erfinderischen Aspekt derart aufeinander abgestimmt, dass die Septumnadel 44 beim Anschlagen des endseitigen Rands 64 an die Stirnfläche 66 noch hinreichend weit vom Septum 28 entfernt positioniert ist und dieses nicht erreicht.

In einer weiteren als eigenständig erfinderisch angesehenen Ausgestaltung ist zwischen dem Grundkörper 50 des Nadelhalters 6 und der Nadelschutzkappe 56 ein rotatorisches Gesperre vorgesehen. Dazu ist in der als eigenständig erfinderisch angesehenen Ausgestaltung gemäß Ausführungsbeispiel außenseitig am Grundkörper 50 des Nadelhalters 6 endseitig eine in dessen Längsrichtung verlaufende Mitnehmerrippe 67 angeformt, die bei aufgeschobener Nadelschutzkappe 56 in eine korrespondierende, innenseitig in die Abdeckschürze 62 eingebrachte Mitnehmernut 68 eingreift, wie dies der vergrößerten Darstellung im Längsschnitt gem. FIG. 4 entnehmbar ist. Durch das durch die Kombination aus Mitnehmerrippe 67 und diese umgreifende Mitnehmernut 68 gebildete Rotationsgesperre ist somit der Grundkörper 50 relativ zur Nadelschutzkappe 56 rotatorisch festgelegt, und mittels einer Drehung der Nadelschutzkappe 56 um ihre Längsrichtung kann der darin liegende Grundkörper 50 des Nadelhalters 6 mitgedreht werden. Selbstverständlich kann ein derrtiges rotatorisches Gesperre zwischen dem Grundkörper 50 und der Nadelschutzkappe 56 auch auf andere Weise dargestellt werden, beispielsweise eine innenseitig an der Abdeckschürze 62 angeformte, in Längsrichtung verlaufende Mitnehmerrippe und eine dazu korrespondierende außenseitig am Grundkörper 50 positionierte Mitnehmernut.

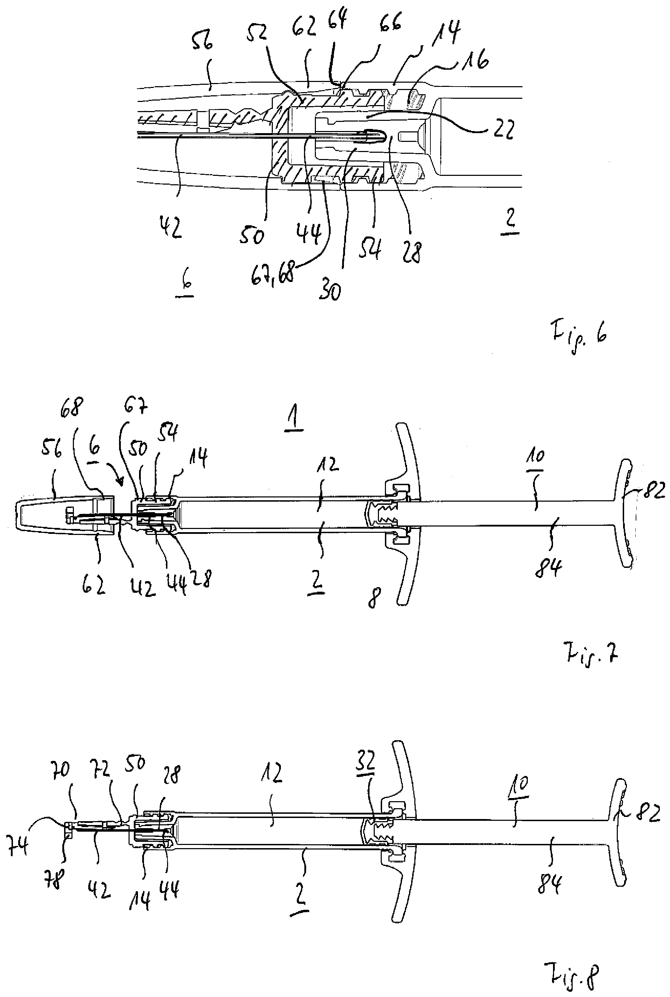

Die insoweit montierte Spritze 1, bei der der mit der aufgeschobenen Nadelschutzkappe 56 versehene Nadelaufsatz 6 bis hin zum genannten Anschlag auf den Spritzenkörper 2 aufgeschraubt ist, ist in FIG. 5 im Längsschnitt und in ausschnittsweiser Vergrößerung in FIG. 6 gezeigt. Deutlich ist dabei erkennbar, dass das Außengewinde 54 mit etwa einem Gewindegang in das Innengewinde 16 eingreift und der endseitige Rand 64 direkt an der Stirnfläche 66 des Anschlussstutzens 14 anliegt. Damit ist eine mechanisch stabile und belastbare Verbindung des Nadelaufsatzes 6 am Spritzenkörper 2 gewährleistet. Ein weiteres Eindrehen des Außengewindes 54 in das Innengewinde 16 hinein ist aufgrund des Anschlags nicht mehr ohne weiteres möglich. Das freie Ende der Septumnadel 44 kann in dieser Position das Septum 28 nicht erreichen, und dieses ist somit vor Beschädigungen durch die Septumnadel 44 geschützt und bleibt intakt. In diesem Zustand ist die vorbefüllte Spritze 1 somit sicher lager- und transportfähig, wobei die Septumnadel 44 zuverlässig "trocken" gehalten wird, d. h. nicht in Kontakt mit dem Wirkstoff im Inneren des Spritzenkörpers 2 treten kann.

Zur Verabreichung des Wirkstoffs, also für den Einsatz der Spritze 1, wird dann vorzugsweise unmittelbar vor der Verwendung die Nadelschutzkappe 56 entfernt, so dass die Injektionsnadel 42 zugänglich gemacht wird. Zu diesem Zweck wird nunmehr der Nadelaufsatz 6 über einen Zugriff auf die Nadelschutzkappe 56 über den vorläufig durch das Rastsystem aus Innennut 58 und Wulst 60 gebildeten Anschlag, durch Anwendung eines geeigneten erhöhten Drehmoments, hinaus weitergedreht, wobei die Drehung über das genannte Rotationsgesperre von der Nadelschutzkappe 56 auf den Grundkörper 50 übertragen wird. Dadurch wird das Außengewinde 54 am Grundkörper 50 weiter in das Innengewinde 16 im Anschlussstutzen 14 eingedreht, wobei die Septumnadel 44 weiter auf das Septum 28 zubewegt wird. Die Nadelschutzkappe 56 kann dabei aufgrund des aus den Rändern 64,66 gebildeten Anschlags nicht in Längsrichtung mitbewegt werden, so dass sie zunehmend vom Grundkörper 50 abgehoben wird und schließlich abgenommen werden kann. Gemeinsam mit der Nadelschutzkappe 56 wird auch deren Abdeckschürze 62 entfernt, so dass der Kontakt ihres endseitigen Rands 64 mit der Stirnfläche 66 des Anschlussstutzens 14 aufgehoben wird. Die Nadelschutzkappe 56 kann in diesem Sinne auch als Originalititätsverschluss angesehen werden, da mit ihrem Entfernen deutlich wird, dass ein Gebrauch des Medikaments bereits möglich war. Die Spritze 1 beim Entfernen der Nadelschutzkappe 56 ist in FIG. 7 im Längsschnitt gezeigt.

Deutlich erkennbar ist dabei, dass in dieser Situation ein weiteres Einschrauben des Außengewindes 54 in das Innengewinde 16 hinein erfolgt ist. Infolge eines solchen weiteren Einschraubens wird durch die Gewindeverbindung ein Vorschub des Nadelaufsatzes 6 in Richtung zum proximalen Ende 8 des Spritzenkörpers 2 erzeugt. Dieser Vorschub transportiert auch die Septumnadel 44 in Richtung zum Septum 28 hin. Ein besonderer Vorteil dieser Anordnung besteht im Übrigen darin, dass durch die Übersetzung der Drehbewegung in eine Vorschubbewegung mittels der Gewindeverbindung, je nach Wahl und Auslegung der Gewindeparameter, ein sehr stabiler, mechanisch gut geführter und sehr fein dosierbarer Vortrieb erzeugt werden kann, so dass die Septumnadel 44 besonders präzise positioniert und bewegt werden kann.

Durch das weitere Einschrauben kann die Septumnadel 44 so weit in Richtung proximales Ende 8 bewegt werden, dass sie das Septum 28 durchstößt und somit die fluidseitige Verbindung der Injektionsnadel 42 mit dem Inneren des Spritzenkörpers 2 und somit des Wirkstoffreservoirs hergestellt werden kann. Die Spritze 1 in diesem, nunmehr gebrauchsfertigen Zustand ist in FIG. 8 im Längsschnitt dargestellt. In diesem Zustand, bei dem die Septumnadel 44 durch die Membran 28 hindurch in das Wirkstoffreservoir hineinragt, kann der Stopfen 32 in Richtung zum distalen Ende 4 und somit zum Verschlusskörper 24 innerhalb des Hohlkörpers 12 verschoben werden, so dass der Wirkstoff durch die Hohlnadel 46 aus dem Innenraum herausgedrückt wird.

Weiterhin ist im Ausführungsbeispiel gemäß einem gerade in Kombination mit den vorstehend beschriebenen Komponenten als eigenständig erfinderisch angesehenen Aspekt der an den Hohlkörper 12 anbringbare Nadelaufsatz 6 oder Nadelhalter mit einem integrierten Nadelschutzsystem versehen. Dabei ist dem Umstand Rechnung getragen, dass es ein zunehmendes Bedürfnis und teilweise auch schon in rechtliche oder regulatorische Vorschriften eingeflossen ist, medizinische Spritzen mit so genannten Nadelschutzsystemen auszurüsten. Zunehmende gesetzliche Vorgaben zur sicheren Arbeitsumgebung in verschiedenen Ländern verpflichten nämlich beispielsweise die Arbeitgeber im medizinischen Bereich zum Schutz ihrer Mitarbeit er vor Nadelstichverletzungen. Dazu kann dann vorgesehen sein, beispielsweise ein System aus Schutzkappen vorzusehen, die über die Nadel gesteckt werden, wenn diese nicht in Benutzung ist, oder es können so genannten Retraktionssysteme vorgesehen sein, bei denen die Nadel nach Gebrauch in das zwischenzeitlich vom Wirkstoff entleerte Spritzengehäuse zurückgezogen wird, so dass die Nadelspitze nicht mehr freiliegt.

Vorliegend ist gemäß einem Aspekt der Erfindung zu diesem Zweck am Grundkörper 50 des Nadelaufsatzes 6 eine Nadelschutzhülle 70 über ein Gelenk 72 schwenkbar angebracht. Die Nadelschutzhülle 70 wird dabei von einem länglich ausgedehnten Schutzkörper 74 gebildet, der in seiner Länge die freie Länge der Injektionsnadel 42 übertrifft und diese somit endseitig überragt. Des Weiteren ist die Breite des Schutzkörpers 74 derart gewählt, dass er die Injektionsnadel 42 beidseitig nach außen hin deutlich überragt. Der Schutzkörper 74 kann zudem innenseitig eine Aufnahmerinne aufweisen, in die die Injektionsnadel 42 in FIG. 8 gezeigten Position des Schutzkörpers 74 eingebettet sein kann. Endseitig ist am Schutzkörper 74 eine Deckplatte 78 angeformt, die in der dort gezeigten Position das freie Ende der Injektionsnadel 42 abdeckt. In der gezeigten Position ist das freie Ende der Injektionsnadel 42 somit vollständig abgeschirmt, und kein Kontakt und somit auch keine Verletzung durch die Hohlnadel 46 ist damit möglich.

In dieser Position befindet sich das System, wenn die äußere Schutzkappe 56 abgenommen wird. Da die Hohlnadel 46 in diesem Zustand noch von dem Schutzkörper 74 abgeschirmt wird, ist noch kein Einstechen der Nadel 46 möglich. Um diese einsetzbar zu machen, wird daher mittels eines am Schutzkörper 74 angeformten Betätigungselements 80 um das Gelenk 72 herum von der Hohlnadel 46 weggeschwenkt, so dass diese nunmehr freigelegt und freigegeben wird. In diesem Zustand ist die Spritze 1 nun zur Abgabe des Wirkstoffs bereit.

Weitere Aspekte zu diesem Nadelschutzsystem sind im Detail in der in der nicht vorveröffentlichten Europäischen Patentanmeldung Nr 22174670.4 ausgeführt, deren Offenbarung bezüglich der Ausgestaltung des Nadelschutzsystems vollumfänglich mit einbezogen wird ("incorporation by reference"). Insbesondere kann der Schutzkörper 74 nach Gebrauch der Spritze 1 um das Gelenk 72 herum wieder zurück geschwenkt werden, so dass er die Injektionsnadel 42 erneut wie vorstehend beschrieben abschirmt und damit Verletzungen durch Berührungen der Nadel 42 sicher vermieden sind. Um aber die Verletzungsgefahr an der dann benutzten Nadel 42 noch weiter zu vermindern, ist gemäß einem weiteren Aspekt der Erfindung vorgesehen, den Schutzkörper 74 um das Gelenk 72 herum noch weiter zu verschwenken, bis die Injektionsnadel 42 in ihrem Nadellager im Grundkörper 50 abbricht. Damit wird die Nadel 42 nunmehr endgültig in ihrer Position innerhalb des Abschirmbereichs des Schutzkörpers 72 fixiert, und zudem wird damit unmittelbar deutlich, dass die Spritze 1 bereits benutzt war und der (nunmehr verbrauchte) Spritzenkörper 2 gemeinsam mit der noch daran montierten, nunmehr zerbrochenen Nadel 42 entsorgt werden soll. Damit sind Irrtümer und Verwechslungen, die beispielsweise zu einem versehentlichen Wiederbenutzen der Spritze 1 führen könnten, sicher ausgeschlossen.

Die Anbringung der genannten Komponenten des Nadelschutzes 70 an der Hohlnadel 46 kann in der Art eines "Overmolding" in einem einzigen, gemeinsamen Arbeitsschritt erfolgen.

Im Ausführungsbeispiel ist ein Betätigungselement 10 gezeigt, das in der Art einer herkömmlichen Ausführung einen an einem Griffstück 82 angeordneten Betätigungsstößel 84 umfasst, der nach der Befestigung des Spritzenkörpers 2 am Griffstück 82 auf den Stopfen 32 wirkt und dessen Verschiebung zum distalen Ende 4 hin ermöglicht. Alternativ kann das Betätigungselement 10 aber auch als gewöhnliches Spritzen-Griffstück oder aber auch als automatisiertes Betätigungselement 10 in der Art eines Autoinjektors ausgeführt sein. Die Verbindung des Betätigungselements 10 mit dem Spritzenkörper 2 kann in unterschiedlichen Variationen ausgeführt sein, beispielsweise als Schraub-, Steck- oder Klickverbindung. Dabei ist dem Umstand Rechnung getragen, dass es ein zunehmendes Bedürfnis und teilweise auch schon in rechtliche oder regulatorische Vorschriften eingeflossen ist, medizinische Spritzen mit so genannten Nadelschutzsystemen auszurüsten. Zunehmende gesetzliche Vorgaben zur sicheren Arbeitsumgebung in verschiedenen Ländern verpflichten nämlich beispielsweise die Arbeitgeber im medizinischen Bereich zum Schutz ihrer Mitarbeit er vor Nadelstichverletzungen. Dazu kann dann vorgesehen sein, beispielsweise ein System aus Schutzkappen vorzusehen, die über die Nadel gesteckt werden, wenn diese nicht in Benutzung ist, oder es können so genannten Retraktionssysteme vorgesehen sein, bei denen die Nadel nach Gebrauch in das zwischenzeitlich vom Wirkstoff entleerte Spritzengehäuse zurückgezogen wird, so dass die Nadelspitze nicht mehr freiliegt.

### Bezugszeichenliste

- 1: Medizinische Spritze
- 2,2': Spritzenkörper
- 4: distales Ende
- 6, 6': Nadelaufsatz
- 8: proximales Ende
- 10: Betätigungselement
- 12: Hohlkörper
- 14: Anschlussstutzen
- 16: Luer-Innengewinde
- 18: Gewinderaum
- 20: Verbindungszapfen
- 22: Verbindungskanal
- 24: Verschlusskörper
- 26: Zentralbereich
- 28: Membran, Septum
- 30: Fixierschürze
- 32: Stopfen
- 34: Stopfenkörper
- 36: Dichtmantel
- 38: Aufnahmekanal
- 40: Nadelsystem
- 42: Injektionsnadel
- 44: Septumnadel
- 46: Hohlnadel
- 50: Grundkörper
- 52: Anschlussbereich
- 54: Außengewinde
- 56: Nadelschutzkappe
- 58: Innennut
- 60: Wulst
- 62: Abdeckschürze
- 64: Rand
- 66: Stirnfläche
- 67: Mitnehmerrippe
- 68: Mitnehmernut
- 70: Nadelschutzhülle
- 72: Gelenk
- 74: Schutzkörper
- 78: Deckplatte
- 80: Betätigungselement
- 82: Griffstück
- 84: Betätigungsstößel

## Patentansprüche

1. Nadelaufsatz (6) mit einem Grundkörper (50), der ein von einer Injektionsnadel (42) und einer mit dieser fluidseitig verbundenen Septumnadel (44) gebildetes Nadelsystem trägt, und der außenseitig mit einem Außengewinde (54) versehen ist, und mit einer Nadelschutzkappe (56), die mittels einer endseitig angeformten Abdeckschürze (62) das Außengewinde (54) teilweise umgibt, zur Verwendung mit einem einen Wirkstoffcontainer aufweisenden Spritzenkörper (2) einer medizinischen Spritze (1), wobei der Wirkstoffcontainer mittels einer im Bereich des distalen Endes (4) des Spritzenkörpers (2) im Spritzenkörper (2) angeordneten, durchstechbaren Membran (28) dichtend verschlossen ist, wobei der Nadelaufsatz (6) derart mittels einer Schraubverbindung am Spritzenkörper (2) anbringbar ist, dass das Einschrauben des Nadelaufsatzes (6) in den Spritzenkörper (2) einen Vortrieb der Septumnadel (44) in Richtung hin zur durchstechbaren Membran (28) bewirkt, und wobei die Abdeckschürze (62) derart angeordnet und dimensioniert ist, dass, wenn ihr endseitiger Rand (64) an der Stirnfläche (66) eines Anschlussstutzens (14) des Spritzenkörpers (2) anliegt, die Septumnadel (44) beabstandet von der Membran (28) im Spritzenkörper (2) ist und ein Durchstechen der Membran (28) durch die Septumnadel (44) erst nach Entfernen der Nadelschutzkappe (62) ermöglicht ist.

2. Medizinische Spritze (1) mit einem einen Wirkstoffcontainer aufweisenden Spritzenkörper (2), wobei der Wirkstoffcontainer mittels einer im Bereich des distalen Endes (4) des Spritzenkörpers (2) im Spritzenkörper (2) angeordneten, durchstechbaren Membran (28) dichtend verschlossen ist, und mit einem am Spritzenkörper (2) anbringbaren Nadelaufsatz (6) nach Anspruch 1.

3. Medizinische Spritze (1) nach Anspruch 2, bei der die Schraubverbindung als Luer-Verbindung ausgestaltet ist.

4. Medizinische Spritze (1) nach einem der Ansprüche 2 oder 3, bei der die Septumnadel (44) die im Spritzenkörper (2) angeordnete Membran (28) durchsticht, wenn die Schraubverbindung vollständig ist.

5. Medizinische Spritze (1) nach einem der Ansprüche 2 bis 4, bei der die Schraubverbindung durch ein an einem Grundkörper (50) des Nadelaufsatzes (6) angeordnetes Außengewinde (54) und ein an dieses angepasstes Innengewinde (14) eines Anschlussstutzens (14) des Spritzenkörpers (2) gebildet ist.

## Claims

1. Needle attachment (6) having a base body (50), which supports a needle system formed from an injection needle (42) and a septum needle (44) connected to the same on the fluid side, and which is provided with an outer thread (54) on the outside, and having a needle protective cover (56) which partially surrounds the outer thread (54) by means of a cover skirt (62) molded onto an end side, for use with a syringe body (2) of a medical syringe (1) having an active ingredient container, wherein the active ingredient container is closed in a sealing way by means of a pierceable membrane (28) arranged in the syringe body (2) in the area of the distal end (4) of the syringe body (2), wherein the needle attachment (6) can be applied to the syringe body (2) by means of a screw connection in such a way that the screwing in of the needle attachment (6) into the syringe body (2) causes the septum needle (44) to be propelled in the direction of the pierceable membrane (28), and wherein the cover skirt (62) is arranged and dimensioned in such a way that when its end-side edge (64) contacts the end face (66) of a connecting piece (14) of the syringe body (2), the septum needle (44) is spaced apart from the membrane (28) in the syringe body (2), and a piercing of the membrane (28) by the septum needle (44) is only enabled after a removal of the needle protective cover (62).

2. Medical syringe (1) having a syringe body (2) having an active substance container, wherein the active substance container is closed in a sealing manner by means of a pierceable membrane (28) arranged in the syringe body (2) in the area of the distal end (4) of the syringe body (2), and having a needle attachment (6) according to claim 1 that can be attached to the syringe body (2).

3. Medical syringe (1) according to claim 2, in which the screw connection is designed as a Luer connection.

4. Medical syringe (1) according to one of claims 2 or 3, in which the septum needle (44) pierces the membrane (28), arranged in the syringe body (2), when the screw connection is completed.

5. Medical syringe (1) according to one of claims 2 to 4, in which the screw connection is formed by an outer thread (54) arranged on a base body (50) of the needle attachment (6) and by an inner thread (14) of a connecting piece (14) of the syringe body (2) adapted to said outer thread.

## Revendications

1. Embout d'aiguille (6), doté d'un corps de base (50), qui porte un système d'aiguilles constitué d'une aiguille d'injection (42) et d'une aiguille pour septum (44) reliée avec celle-ci côté fluide et qui sur la face extérieure, est muni d'un filetage (54) et doté d'un capuchon protecteur (56) d'aiguille, qui au moyen d'une jupe de recouvrement (62) surmoulée sur le côté extrémité, entoure partiellement le filetage (54), destiné à l'utilisation avec un corps de seringue (2) comportant un réservoir de principe actif d'une seringue médicale (1), le réservoir de principe actif étant fermé de manière à assurer l'étanchéité au moyen d'une membrane (28) perçable, placée dans le corps de seringue (2), dans la zone de l'extrémité distale (4) du corps de seringue (2), l'embout d'aiguille (6) étant susceptible d'être monté sur le corps de seringue (2) au moyen d'un raccord vissé, de telle sorte que le vissage de l'embout d'aiguille (6) dans le corps de seringue (2) provoque une propulsion de l'aiguille pour septum (44) dans la direction vers la membrane (28) perçable et la jupe de recouvrement (62) étant placée et dimensionnée de telle sorte, que lorsque son bord (64) côté extrémité est adjacent à la face frontale (66) d'une tubulure de raccordement (14) du corps de seringue (2), l'aiguille pour septum (44) soit écartée de la membrane (28) dans le corps de seringue (2) et qu'un perçage de la membrane (28) par l'aiguille pour septum (44) ne soit possible qu'une fois que la capuchon protecteur (62) d'aiguille a été retiré.

2. Seringue médicale (1), dotée d'un corps de seringue (2) comportant un réservoir de principe actif, le réservoir de principe actif étant fermé de manière à assurer l'étanchéité au moyen d'une membrane (28) perçable, placée dans le corps de seringue (2) dans la zone de l'extrémité distale (4) du corps de seringue (2) et dotée d'un embout d'aiguille (6) selon la revendication 1, susceptible d'être monté sur le corps de seringue (2).

3. Seringue médicale (1) selon la revendication 2, sur laquelle le raccord vissé est conçu sous la forme d'un raccord Luer.

4. Seringue médicale (1) selon l'une quelconque des revendications 2 ou 3, sur laquelle l'aiguille pour septum (44) perce la membrane (28) placée dans le corps de seringue (2) lorsque le raccord vissé est intégral.

5. Seringue médicale (1) selon l'une quelconque des revendications 2 à 4, sur laquelle le raccord vissé est constitué par un filetage (54) placé sur le corps de base (50) de l'embout d'aiguille (6) et par un taraudage (14) adapté à celui-ci d'une tubulure de raccordement (14) du corps de seringue (2).
